# EUROPÄISCHE PATENTSCHRIFT

(11) **EP 0 373 734 B1**
(45) Veröffentlichungstag und Bekanntmachung des Hinweises auf die Patenterteilung: **09.02.1994**
(21) Anmeldenummer: 89250104.0
(22) Anmeldetag: 04.12.1989
(51) Int. Cl.: A61C 8/00

(54) **Enossales Implantat**
Endosseous implant
Implant endo-osseux

(30) Priorität: 10.12.1988 DE 3841705
(43) Veröffentlichungstag der Anmeldung: 20.06.1990
(73) Patentinhaber: IMZ-Fertigungs- und Vertriebsgesellschaft für dentale Technologie mbH, 70794 Filderstadt (DE)
(72) Erfinder: Kirsch, Axel, Dr., D-7024 Filderstadt (DE)
(74) Vertreter: Goddar, Heinz J., Dr.

(56) Entgegenhaltungen:
- EP-A- 0 158 333
- EP-A- 0 202 908
- FR-A- 2 350 090
- GB-A- 2 063 680
- US-A- 4 645 453
- US-A- 4 657 510

## Beschreibung

Die Erfindung betrifft ein enossales Implantat mit den Merkmalen des einleitenden Teiles des Patentanspruches 1.

Enossale Implantate mit einem implantierbaren Grundkörper und einem damit verbindbaren Implantatpfosten sind bereits in verschiedenen Ausführungsformen bekannt. Sie dienen entweder zur Befestigung von Einzelzahnprothesen oder als Trägerkonstruktion für Brücken oder dergleichen.

Als besonders vorteilhaft hat sich auf diesem Gebiet ein enossales Implantat erwiesen, wie es Gegenstand der EP-A-0 216 031 ist. Dieses Implantat weist ebenfalls einen in den Kieferknochen implantierbaren Grundkörper und einen damit verbindbaren Implantatpfosten auf. Bedauerlicherweise kommt es hin und wieder bei besonders extremen Belastungen vor, daß das Implantat an seiner statisch schwächsten Stelle, d. h. im Übergangsbereich des Implantatpfostens zwischen Grundkörper und Zahnprothese, bricht. Bei einem derartigen Abbrechen des Implantatpfosten sind bei den bisher bekannten Ausführungsformen von enossalen Implantaten diese in ihrer Gesamtheit verloren. In einem solchen Fall ist daher eine sehr schmerzhafte und langwierige Extraktion des Grundkörpers aus dem Kieferknochen erforderlich.

Aus der US-A-4 657 510 ist bereits ein Implantat der eingangs genannten Art bekannt, bei dem die Distanzhülse beispielsweise aus Metall besteht. Dabei kann es aufgrund des sauren Speichelmilieus zur Ausbildung von galvanischen Lokalelementen im Grenzbereich zwischen Grundkörper und/oder Distanzhülse auf der einen Seite und der Distanzhülse auf der anderen Seite kommen.

Der Erfindung liegt die Aufgabe zugrunde, das bekannte enossale Implantat dahingehend weiterzubilden, daß es aufgrund des sauren Speichelmilieus nicht zur Ausbildung von galvanischen Lokalelementen im Grenzbereich zwischen Grundkörper und/oder Innenhülse auf der einen Seite und Distanzhülse auf der anderen Seite kommen kann.

Erfindungsgemäß wird diese Aufgabe durch die im kennzeichnenden Teil des Patentanspruches 1 genannten Merkmale gelöst.

Besondere Ausführungsformen der Erfindung sind Gegenstand des Unteransprüche.

Wenn bei dem Implantat nach der Erfindung der Implantatpfosten abbricht, ist es problemlos möglich, diesen durch ein Herausschrauben der Innenhülse zu entfernen. Der implantierte und in vielen Fällen bereits knöchern eingewachsene Grundkörper muß dabei nicht aus dem Kieferknochen entfernt werden. Vielmehr kann durch Einsatz einer neuen Innenhülse und eines intakten Implantatpfostens das Implantat insgesamt problemlos ersetzt werden.

Um die Innenhülse möglichst einfach und schnell aus dem Grundkörper entfernen zu können, sind dabei vorteilhafterweise in ihrem oberen Abschnitt Blindbohrungen für den Eingriff eines entsprechenden Spezialschlüssels vorgesehen. Für das erstmalige Einsetzen der Innenhülse in den Grundköper oder deren Ausbau in dem Fall, daß der Bruch des Implantatpfostens innerhalb der Innenhülse aufgetreten ist, ist erfindungsgemäß vorgesehen, daß der obere Abschitt der Innenhülse einen mehreckigen, bevorzugt sechseckigen Innenquerschnitt aufweist. Auf diese Weise ist der Ein- und Ausbau der Innenhülse auf besonders einfache Weise mit einem Imbusschlüssel möglich.

Dadurch, daß bei dem Implantat nach der Erfindung die Innenhülse aus demselben Material wie der Grundkörper besteht, kann einmal vermieden werden, daß es aufgrund verschiedener Wärmeausdehnungskoeffizienten zu einer Lockerung der Verbindung zwischen Grundkörper und Innenhülse kommt, zum anderen wird hierdurch auch verhindert, daß, wie es beispielsweise bei Verwendung von zwei verschiedenen Metallen der Fall ist, innere Lokalelemente im Implantat entstehen.

Erfindungsgemäß kann vorgesehen sein, daß der Grundkörper an seinem äußeren Umfang mindestens eine den Grundkörper nach seinem unteren Ende zu verjüngende Abstufung aufweist. Diese sich nach unten verjüngende Form des Implantats ist auf diese Weise in natürlicher Form einer Zahnwurzel angepaßt und läßt sich daher einfacher in das Extraktionsloch einpassen. Gleichzeitig können durch das Vorsehen weiterer äußerer Abstufungen Grundkörper mit verschiedenen Durchmessern an ihrem oberen Rand zur Verfügung gestellt werden, was ebenfalls notwendig ist, da auch der obere Durchmesser der Extraktionslöcher je nach Patient bzw. Zahnart variiert. Darüber hinaus bewirken die äußeren Abstufungen des Grundkörpers, daß dieser formschlüssig knöchern einwachsen kann.

Demselben Zweck dienen auch die bevorzugt vorgesehenen Lakonen an der äußeren Umfangsfläche des Grundkörpers. Dabei handelt es sich um ggf. unregelmäßig verteilte Vertiefungen, die beim Einheilen des Grundkörpers ebenfalls in Eingriff mit dem Kieferknochen kommen können und so einen sichereren Sitz des Implantats gewährleisten.

Ein besonderer Vorteil der erfindungsgemäßen Distanzhülse besteht darin, daß diese den Grundkörper, der zunächst unter der sich wieder schließenden Schleimhaut in den Kieferknochen einheilt, starr bis über die Oberkante der Schleimhaut verlängert, ohne daß diese durch Verformungsbewegungen gereizt wird. Im Gegensatz zum eingangs genannten Stand der Technik ist beim Implantat nach der Erfindung eine mit Flansch versehene Innenhülse vorgesehen, deren Flansch das Distanzelement an den Grundkörper andrückt. Hierdurch ist es möglich, die gesamte Distanzhülse aus elektrisch isolierendem Keramikmaterial herzustellen, weil diese Art der Befestigung der Distanzhülse keine allzu hohe mechanische Beanspruchung bewirkt, wie sie beispielsweise bei einer Befestigung nach Art der US-A-4 872 839 zu besorgen wäre. In der zuletzt genannten Druckschrift ist ausdrücklich darauf hingewiesen, daß das Distanzelement dort nicht vollständig aus Keramikmaterial bestehen kann, im Gegensatz zum Implantat nach der Erfindung.

Durch das Übergreifen des Flansches oder mindestens eines Vorsprungs am oberen Rand der Innenhülse auf die Distanzhülse wird ein fester, sicherer Sitz derselben gewährleistet, ohne daß hierfür eine besondere Formanpassung zwischen Grundkörper und Distanzhülse nötig wäre. Infolge der Materialauswahl für die Distanzhülse aus elektrisch isolierendem Material kann es nicht aufgrund des sauren Speichelmilieus zur Ausbildung von Lokalelementen im Grenzbereich zwischen Grundkörper und/oder Innenhülse auf der einen Seite und Distanzhülse auf der anderen Seite kommen.

Der mit dem Grundkörper unter Zwischenschaltung der Innenhülse verbindbare Implantatpfosten ist bevorzugt als elastisch verformbares Element aus Kunststoff ausgebildet. Hierdurch wird das Risiko von Relativbewegungen zwischen Zahnersatz und Grundkörper reduziert, was allerdings, wie bereits zuvor erwähnt, bei bekannten Ausführungsformen dazu geführt hat, daß Implantatpfosten und Grundkörper, insbesondere nach einem Abbrechen des Implantatpfostens, kaum mehr voneinander zu trennen waren. Dieses Problem tritt beim erfindungsgemäßen Implantat wegen der Zwischenschaltung der starren, aus demselben Material wie der Grundkörper bestehenden Innenhülse nicht auf.

Weitere Einzelheiten und Vorteile der Erfindung werden nachstehend anhand der Zeichnungen noch näher erläutert.

Dabei zeigen:
- Fig. 1: einen Längsschnitt einer bevorzugten Ausführungsform eines erfindungsgemäßen Implantats;
- Fig. 2: einen Längsschnitt durch eine abgewandelte Ausführungsform eines erfindungsgemäßen Implantats; und
- Fig. 3: einen Querschnitt durch den oberen Randbereich einer Innenhülse gemäß Fig. 2.

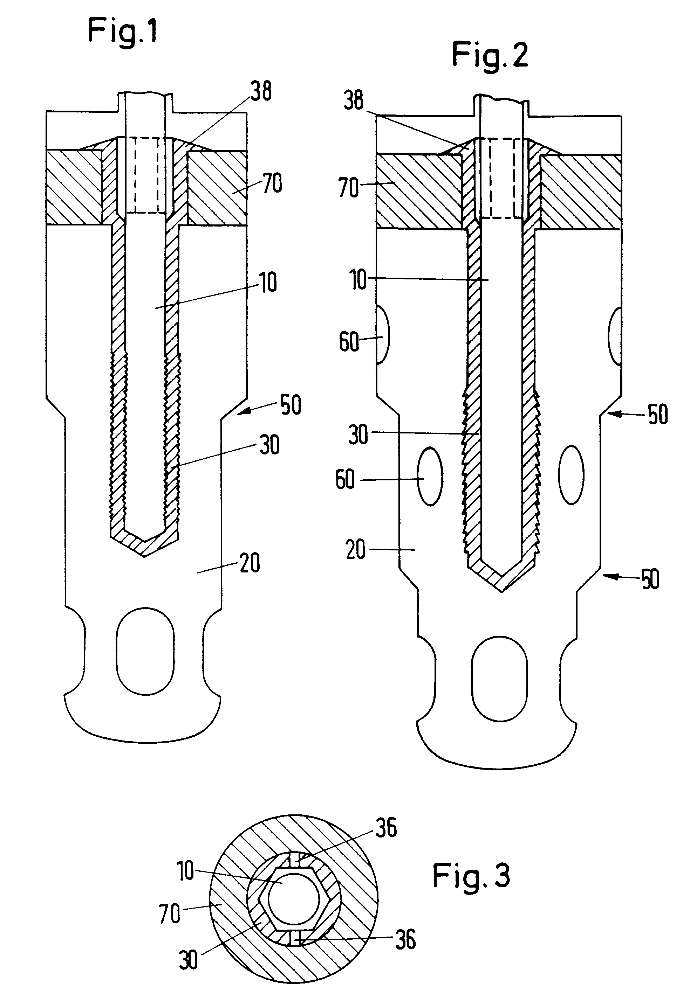

Im einzelnen zeigt Fig. 1 ein erfindungsgemäßes Implantat mit einem Grundkörper 20, der bei der Behandlung eines Patienten in eine vorbereitete Bohrung im Kieferknochen bzw. in ein dort vorhandenes Extraktionsloch paßgenau eingesetzt wird und dort innerhalb eines Zeitraums von etwa drei Monaten knöchern einheilt. Der Grundkörper 20 besteht aus Titan oder einer Titanlegierung und ist auf seiner Außenfläche entweder durch Rändeln oder Sandstrahlen aufgerauht oder in der bereits oben geschilderten Art und Weise gewebefreundlich beschichtet.

Der Grundkörper 20 ist nach oben offen und mit einem Innengewinde versehen, in das eine Innenhülse 30 mit einem in das Innengewinde des Grundkörpers eingreifenden Außengewinde eingeschraubt ist.

Auf den oberen Rand des Grundkörpers 20 ist eine Distanzhülse 70 mit dem gleichen Außendurchmesser wie der Grundkörper aufgesetzt. Auf dem oberen Rand dieser Distanzhülse 70 stützt sich die eingeschraubte Innenhülse 30 mittels eines Ringflansches 38 ab.

Der Implantatpfosten 10, der aus einem elastischen, verformbaren Kunststoff besteht, ist schließlich mit der Innenhülse verbunden, in diesem Fall ebenfalls verschraubt.

Der Grundkörper selbst verjüngt sich nach unten zu durch eine in seinem oberen Drittel vorgesehene Abstufung 50. Im unteren Drittel des Grundkörpers 20, in den die Innenhülse 30 nicht mehr eingreift, sind durchgehende Aussparungen vorgesehen, um den Verwachsungsprozeß mit dem Kieferknochen zu begünstigen.

Bei dem Ausführungsbeispiel gemäß Fig. 2 ist gegenüber Fig. 1 eine weitere Abstufung 50 im unteren Drittel des Grundkörpers 20 vorgesehen. Schließlich sind im oberen Drittel dieses Ausführungsbeispiels nicht-durchgehende Vertiefungen, sogenannte Lakonen 60, zur Begünstigung des knöchernen Einheilens des Implantats vorgesehen. Der Implantatpfosten 10 ist in diesem Fall in der Innenhülse 30 einzementiert.

Fig. 3 schließlich zeigt einen Querschnitt durch den oberen Randbereich von Fig. 2 (oder auch Fig. 1). In dieser Darstellung werden zum einen der sechseckige Innenquerschnitt der Innenhülse 30 als zum anderen auch die in diesem Bereich angebrachten Blindbohrungen 36 deutlich, die den Eingriff eines Imbusschlüssels bzw. eines entsprechenden Spezialschlüssels ermöglichen, um einen raschen und einfachen Ein- und Ausbau der Innenhülse in den Grundkörper zu gewährleisten.

## Patentansprüche

1. Enossales Implantat, mit einem implantierbaren Grundkorper (20), mit mit Innengewinde versehener axialer Innenbohrung und einer starren, mit einem Außengewinde versehenen Innenhülse (30), die lösbar in die Innenbohrung des Grundkörpers eingeschraubt ist und eine axial verlaufende Bohrung aufweist, in der ein Implantatpfosten (10) aufgenommen ist, wobei die axiale Bohrung der Innenhülse einen oberen Abschnitt mit Ansetzeinrichtungen für einen Schlüssel aufweist, der Grundkörper und die Innenhülse aus ein und demselben Metall aus der Gruppe Titan und Titanlegierungen hergestellt sind und der Grundkörper an seinem distalen Ende durch eine Distanzhülse (70) verlängert ist, die auf den distalen Rand des Grundkörpers aufsetzbar ist, dadurch gekennzeichnet, daß die Innenhülse (30) an ihrem distalen Rand einen Ringflansch (38) aufweist, der sich auf der Distanzhülse (70) abstützt und diese auf dem Grundkörper (20) hält; daß die Distanzhülse (70) denselben Außendurchmesser wie der Grundkörper (20) an seinem oberen Rand aufweist; und daß die Distanzhülse aus elektrisch isolierendem Material, ausgewählt aus Aluminiumoxid, Magnesiumoxid und/oder Zirkoniumdioxid besteht.

2. Implantat nach Anspruch 1, dadurch gekennzeichnet, daß die Innenhülse in ihrem oberen Abschnitt Blindbohrungen (36) aufweist.

3. Implantat nach Anspruch 1 oder 2, dadurch gekennzeichnet, daß die Innenhülse (30) in ihrem oberen Abschnitt einen mehreckigen Innenquerschnitt aufweist.

4. Implantat nach Anspruch 3, dadurch gekennzeichnet, daß die Innenhülse (30) in ihrem oberen Abschnitt einen sechseckigen Innenquerschnitt aufweist.

5. Implantat nach einem der vorangehenden Ansprüche, dadurch gekennzeichnet, daß der Grundkörper (20) an seinem äußeren Umfang mindestens eine den Grundkörper nach seinem unteren Ende zu verjüngende Abstufung (50) aufweist.

6. Implantat nach einem der vorangehenden Ansprüche, dadurch gekennzeichnet, daß der Grundkörper (20) über seine äußere Umfangsfläche verteilt Lakonen (60) aufweist.

7. Implantat nach einem der vorangehenden Ansprüche, dadurch gekennzeichnet, daß der Grundkörper (20) von außen gewebefreundlich beschichtet ist.

8. Implantat nach Anspruch 7, dadurch gekennzeichnet, daß auf die Außenfläche des Grundkörpers (20) eine Plasmabeschichtung aus Hydroxylapatit aufgebracht ist.

9. Implantat nach einem der vorangehenden Ansprüche, dadurch gekennzeichnet, daß der Implantatpfosten (10) als elastisch verformbares Element aus Kunststoff ausgebildet ist.

10. Implantat nach einem der vorangehenden Ansprüche, dadurch gekennzeichnet, daß der Implantatpfosten (10) mit einem Außengewinde versehen ist, welches mit einem Innengewinde der Innenhülse (30) verschraubt ist.

11. Implantat nach einem der Ansprüche 1 bis 9, dadurch gekennzeichnet, daß der Implantatpfosten (10) mit der Innenhülse (30) verklebt ist.

12. Implantat nach einem der Ansprüche 1 bis 9, dadurch gekennzeichnet, daß der Implantatpfosten (10) in der Innenhülse (30) einzementiert ist.

## Claims

1. An endosseous implant, comprising an implantable foundation member (20) having an internally screwthreaded axial inner bore and a rigid externally screwthreaded inner sleeve (30) which is releasably screwed into the inner bore of the foundation member and has an axially extending bore in which an implant post (10) is received, the axial bore of the inner sleeve comprising an upper portion having attachment means for a key, the foundation member and the inner sleeve being made from one and the same metal of the group comprising titanium and titanium alloys and the foundation member being lengthened, at its distal end, by a spacer sleeve (70), which is adapted to be fitted on to the distal edge of the foundation member, characterised in that the inner sleeve (30) has at its distal edge an annular flange (38) which bears on the spacer sleeve (70) and holds the latter on the foundation member (20); in that the spacer sleeve (70) has the same outside diameter as the foundation member (20) at its top edge; and in that the spacer sleeve consists of electrically insulating material selected from aluminium oxide, magnesium oxide and/or zirconium dioxide.

2. An implant according to claim 1, characterised in that the inner sleeve has blind bores (36) in its top portion.

3. An implant according to claim 1 or 2, characterised in that the inner sleeve (30) has a polygonal internal cross-section in its top portion.

4. An implant according to claim 3, characterised in that the inner sleeve (30) has a hexagonal internal cross-section in its top portion.

5. An implant according to any one of the preceding claims, characterised in that the foundation member (20) has, at its outer periphery, at least one step (50) resulting in the foundation member tapering towards its bottom end.

6. An implant according to any one of the preceding claims, characterised in that the foundation member (20) has recesses (60) distributed over its outer peripheral surface.

7. An implant according to any one of the preceding claims, characterised in that the foundation member (20) is coated so as to be externally tissue-friendly.

8. An implant according to claim 7, characterised in that a plasma coating of hydroxyl apatite is applied to the outer surface of the foundation member (20).

9. An implant according to any one of the preceding claims, characterised in that the implant post (10) is constructed as an elastically deformable element of plastic.

10. An implant according to any one of the preceding claims, characterised in that the implant post (10) is provided with an external screwthread which is screwed to an internal screwthread of the inner sleeve (30).

11. An implant according to any one of claims 1 to 9, characterised in that the implant post (10) is glued to the inner sleeve (30).

12. An implant according to any one of claims 1 to 9, characterised in that the implant post (10) is cemented in the inner sleeve (30).

## Revendications

1. Implant endo-osseux comprenant un corps de base (20) pouvant être implanté et comportant un alésage intérieur axial pourvu d'un filetage intérieur ainsi qu'une douille intérieure (30) rigide pourvue d'un filetage extérieur, qui est vissée de manière amovible dans l'alésage intérieur du corps de base et qui comporte un alésage d'étendue axiale, dans lequel est logé un pivot d'implant (10), l'alésage axial de la douille intérieure présentant un tronçon supérieur comportant des dispositifs de prise pour une clé, le corps de base et la douille intérieure étant fabriqués dans un seul et même métal du groupe titane et alliages de titane, et le corps de base étant prolongé à son extrémité distale par une douille d'espacement (70) qui peut être appliquée sur le bord distal du corps de base, caractérisé en ce que la douille intérieure (30) comporte à son extrémité distale, un flasque annulaire (38) qui s'appuie sur la douille d'espacement (70) et maintient celle-ci sur le corps de base, en ce que la douille d'espacement (70) présente un diametre extérieur identique à celui du corps de base (20) à son bord supérieur, et en ce que la douille d'espacement est réalisée en un matériau électriquement isolant, choisi entre l'oxyde d'aluminium, l'oxyde de magnésium et/ou l'oxyde de zirconium.

2. Implant selon la revendication 1, caractérisé en ce que la douille intérieure comporte des perçages borgnes 36 dans son tronçon supérieur.

3. Implant selon la revendication 1 ou 2, caractérisé en ce que la douille intérieure (30) présente, dans son tronçon supérieur, une section transversale intérieure polygonale.

4. Implant selon la revendication 3, caractérisé en ce que la douille intérieure (30) présente, dans son tronçon supérieur, une section transversale intérieure de forme hexagonale.

5. Implant selon l'une des revendications précédentes, caractérisé en ce que le corps de base (20) comporte sur sa périphérie extérieure, au moins un étagement (50) rétrécissant le corps de base en direction de son extrémité inférieure.

6. Implant selon l'une des revendications précédentes, caractérisé en ce que le corps de base (20) comporte des lacunes (60) réparties sur sa surface périphérique extérieure.

7. Implant selon l'une des revendications précédentes, caractérisé en ce que le corps de base (20) présente extérieurement un revêtement de bonne compatibilité tissulaire.

8. Implant selon la revendication 7, caractérisé en ce que sur la surface extérieure du corps de base (20) est appliquée une couche d'hydroxyapatite.

9. Implant salon l'une des revendications précédentes, caractérisé en ce que le pivot d'implant (10) est réalisé en tant qu'élément déformable de manière élastique, en matière plastique.

10. Implant selon l'une des revendications précédentes, caractérisé en ce que le pivot d'implant (10) est pourvu d'un filetage extérieur qui est vissé dans le filetage intérieur de la douille intérieure (30).

11. Implant selon l'une des revendications 1 à 9, caractérisé en ce que le pivot d'implant (10) est fixé par collage sur la douille intérieure (30).

12. Implant selon l'une des revendications 1 à 9, caractérisé en ce que le pivot d'implant (10) est cimenté dans la douille intérieure (30).
